# EUROPEAN PATENT SPECIFICATION

(11) **EP 0 643 073 B1**
(45) Date of publication and mention of the grant of the patent: **26.11.1997**
(21) Application number: 94306667.0
(22) Date of filing: 12.09.1994
(51) Int. Cl.: C07K 5/08

(54) **Tripeptide antithrombotic agents**
Tripeptid mit antithrombischer Wirkung
Agents antithrombotiques à base de tripeptides

(30) Priority: 14.09.1993 US 121134
(43) Date of publication of application: 15.03.1995
(73) Proprietor: ELI LILLY AND COMPANY, Indianapolis, Indiana 46285 (US)
(72) Inventor: Gesellchen, Paul David, Indianapolis, Indiana 46240 (US); Shuman, Robert Theodore, Greenwood, Indiana 46143 (US)
(74) Representative: Hudson, Christopher Mark

(56) References cited:
- EP-A- 0 479 489

## Description

This invention relates to thrombin inhibitors which are useful anticoagulants in humans and animals. In particular it relates to derivatives of the dipeptide L-Proline-L-Arginine aldehyde having high antithrombotic activity.

Thrombin inhibition is currently achieved by the administration of heparins and coumarins. The mechanism by which these agents act has been much studied. Heparins are only administerable parenterally and levels must be carefully monitored. Coumarins act by blocking or inhibiting the formation of prothrombin and require some time to achieve maximum effectiveness.

Although both the heparins and the coumarins are effective anticoagulants, there exists a need for antithrombin agents which act quickly to prevent clot formation and which do not interfere with plasmin action in dissolving existing clots.

The present invention is directed to the unexpected discovery that the compound of the present invention, is considerably more potent as a thrombin inhibitor than the corresponding 4aS, 8aS enantiomer.

Accordingly, it is a primary object of the present invention to provide (1R, 4aR, 8aR)-1,2,3,4,5,6,7,8-perhydroisoquinolin-1-carbonyl-(L)-prolinyl-(L)-arginine aldehyde and pharmaceutically acceptable salts and solvates thereof that is a surprisingly more potent thrombin inhibitor and is useful as an anticoagulant and thromboembolic disorder agent.

The thrombin inhibiting compound provided by this invention is represented by the following formula 1. 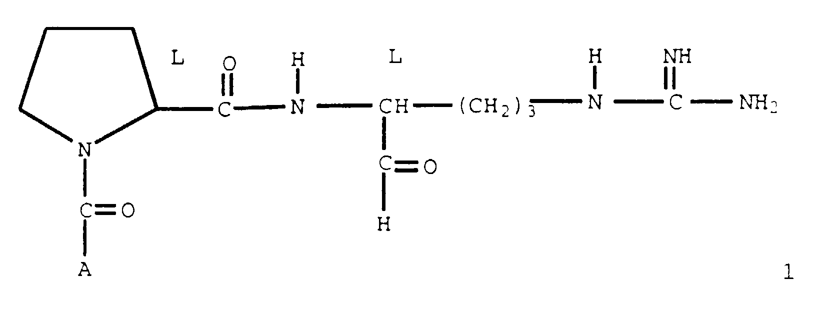 wherein A is 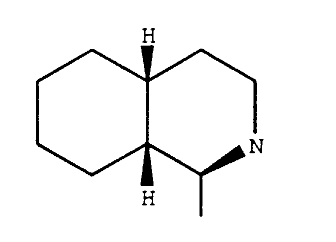 and the pharmaceutically acceptable non-toxic salts and solvates thereof.

The peptide represented by the formula 1 is a useful antithrombotic agent and can be used as an adjunct to tissue plasminogen activator (tPA), streptokinase or urokinase therapy.

The compound is prepared by conventional coupling methods as disclosed in EP O 479 489 A2; U.S. 5,250,660; and U.S. 5,252,566. For example, Cbz-1,2,3,4,5,6,7,8-perhydro-1-isoquinolincarboxylic acid is coupled with an ester of L-proline to form Cbz-1,2,3,4,5,6,7,8-perhydroisoquinolin-1-carbonyl-Pro ester. The ester group is removed and the Cbz-1,2,3,4,5,6,7,8-perhydroisoquinolin-1-carbonyl-Pro is coupled with the lactam form of L-arginine to provide Cbz-1,2,3,4,5,6,7,8-perhydroisoquinolin-1-carbonyl-Pro-Arg lactam in amino protected form. The Arg lactam ring is opened by reduction and the arginine amino and perhydroisoquinoline nitrogen protecting groups removed to provide 1,2,3,4,5,6,7,8-perhydroisoquinolin-1-carbonyl-Pro-Arg aldehyde. The peptide is converted to suitable salt forms such as the acetates and sulfates.

1,2,3,4,5,6,7,8-perhydro-1-isoquinolincarboxylic acid is readily prepared by hydrogenation of 1-isoquinolincarboxylic acid in ethanol or other suitable alcohol in the presence of 5N hydrochloric acid, or other suitable strong inorganic acid, over five percent Rh/Al₂O₃ or other suitable catalyst at a pressure from about 500 to about 1000 psi and a temperature from about 30°C to about 80°C.

This procedure affords (1R, 4aS, 8aS)-1,2,3,4,5,6,7,8-perhydro-1-isoquinolincarboxylic 8aR)-1,2,3,4,5,6,7,8-perhydro-1-isoquinolincarboxylic acid and (1S, 4aR, acid as a racemic mixture. The individual enantiomers can be separated by resolution of the racemate by classical methods. Such methods include the formation of salts with optically active acids and also by high pressure liquid chromotography of the racemate over chiral columes.

The thermodynamic isomers (1R, 4aR, 8aR)-1,2,3,4,5,6,7,8-perhydro-1-isoquinolincarboxylic acid and (1S, 4aS, 8aS)-1,2,3,4,5,6,7,8-perhydro-1-isoquinolincarboxylic acid are afforded by preparing the esters of the racemic mixture afforded by the above procedures and reacting those esters with sodium ethoxide in ethanol and then deesterifying the resulting racemic mixture of thermodynamic isomers.

Alternatively, the racemic mixture of isomers and the racemic mixtures of thermodynamic isomers, as the acids are amino protected and coupled with a carboxy protected proline to afford the dipeptide. The diastereomers resulting from the coupling with L-proline are then resolved by crystallization.

The carboxy protecting ester group of the proline moiety of the dipeptide is then removed (deblocked or deesterified) and the free acid form of the dipeptide is coupled with the lactam form of arginine. The coupled arginine (amino-protected) lactam product is reacted with a hydride reducing agent, preferably lithium aluminum hydride or lithium tri-tert-butoxyaluminohydride in an inert solvant or mixture of solvants to reduce the lactam ring and provide the tripeptide in the arginine aldehyde form. The protecting groups are then removed by procedures known to those skilled in the art such as hydrogenation over a metal catalyst.

The invention also provides a method for preventing the formation of clots in mammals and pharmaceutical formulations useful in the method.

As shown in formula 1, the asymmetric center of the proline and arginine aldehyde moieties is L.

The perhydroderivatives including D-1,2,3,4,4a,5,6,7,8,8a-decahydroisoquinolin-1-carbonyl (perhydroisoquinolin-1-carbonyl or 1-Piq) and D-1,2,3,4,4a,5,6,7,8,8a-decahydroisoquinolin-3-carbonyl (perhydroisoquinolin-3-carbonyl or 3-Piq) derivatives of Pro-Arg-H depicted below.

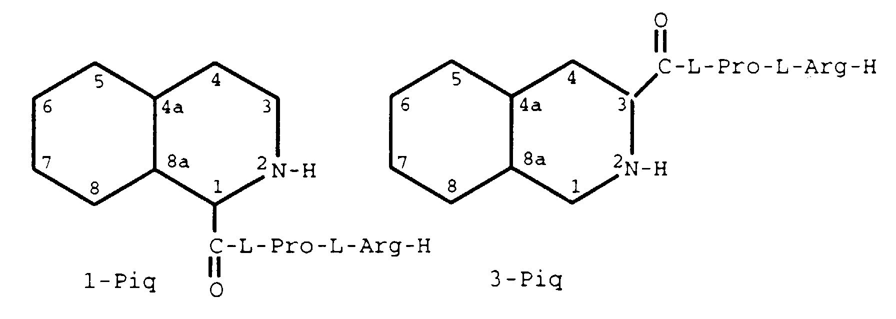

The perhydro derivatives can exist as cis or trans stereoisomers. For example, the following pair of stereoisomers identified as cis can be formed for D-perhydroisoquinolin-1-carbonyl-L-prolyl-L-arginine aldehyde:

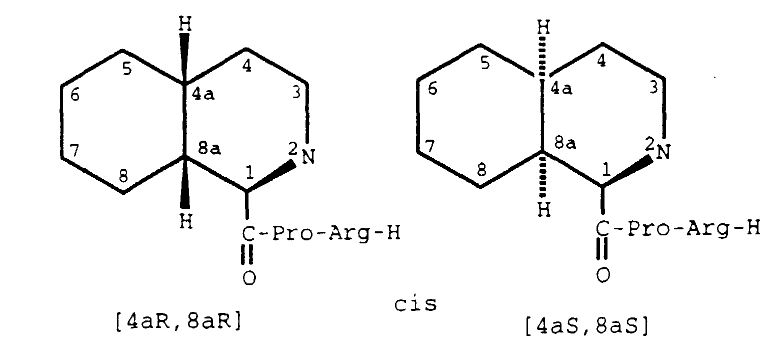

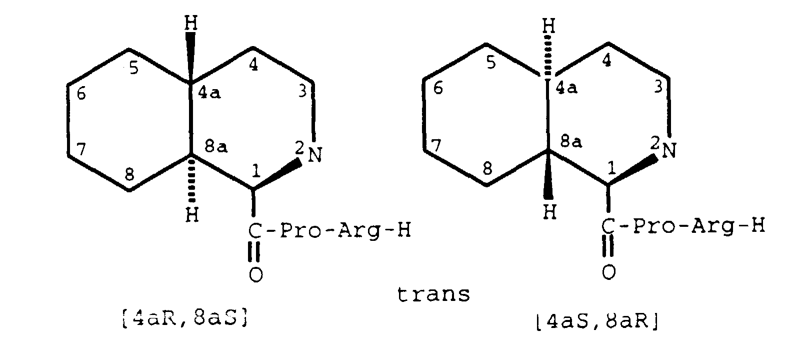

Pharmaceutically acceptable salts of peptides of the invention include the acid addition salts formed with inorganic acids and carboxylic acids. Examples of inorganic acids forming salts are the hydrohalic acids hydrochloric and hydrobromic; phosphoric acid and sulfuric acid. Carboxylic acid salts are formed with acids such as acetic, propionic, malonic, maleic, citric, succinic, malic, benzoic, fumaric, and like carboxylic acids. The acid addition salts are prepared in a conventional manner e.g. by neutralizing the free base form of the compound 1 with the acid. Preferred acid addition salts are sulfate hydrochloride salts.

As stated above, the present invention includes solvates of the compound of this invention and its pharmaceutically acceptable salts. The compound of the present invention or a pharmaceutically acceptable salt thereof may form solvates with water or common organic solvents. Such solvates are included within the scope of the present invention.

The compound represented by the formula 1 is prepared by known methods of peptide coupling. According to one such method, the acid A-COOH, wherein A has the same meaning as defined for formula 1, and the nitrogen atom is protected with a suitable amino protecting group, is coupled with a carboxy protected proline to form the dipeptide. The carboxy protecting ester group of the proline moiety of the product is removed and the free acid form of the dipeptide is coupled with the lactam form of arginine. The above reaction sequence is illustrated by the following scheme. 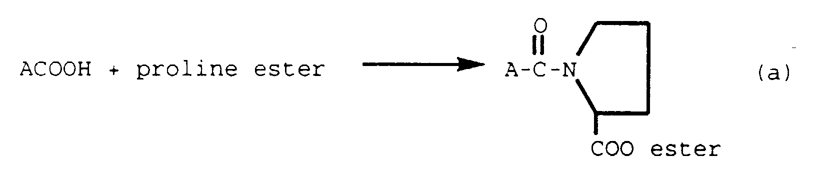 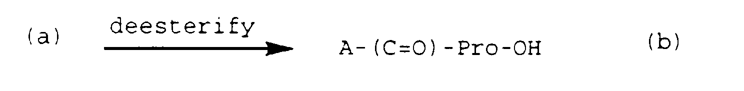 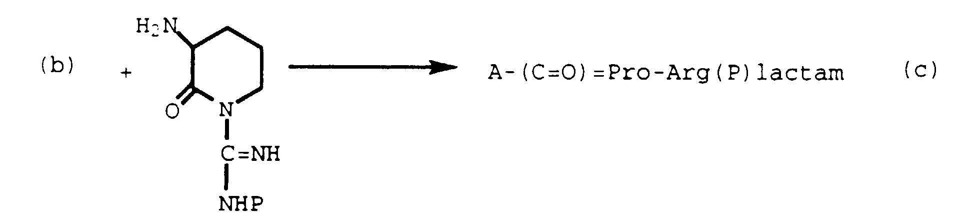 wherein P represents an amino protecting group.

The coupled Arg(P) lactam product (c) is reduced with lithium aluminum hydride in an inert solvent to cleave the lactam ring and provide the tripeptide in the arginine aldehyde form represented by the formula

A(C=O)-Pro-Arg(P)-H

wherein Arg(P)-H represents amino protected arginine aldehyde.

The lactam form of arginine is obtained by intramolecular coupling of amino protected arginine [Arg-OH]. For example, Boc-Arg(Cbz)OH represented by the formula 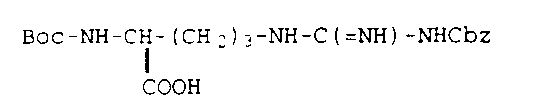 is first converted to an active ester form, such as an active mixed anhydride, with a chloroformate ester, e.g., ethyl chloroformate to isobutyl chloroformate. The ester formation is carried out in the present of a tertiary amine such as N-methylmorpholine. Addition of a stronger tertiary amine base such as triethylamine effects the internal acylation to provide the lactam form of the diamino protected arginine as shown below. 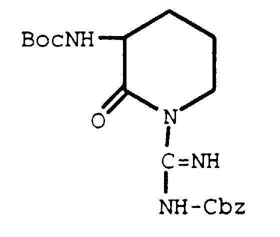 Prior to use in the coupling with the A(C=O)-Pro-OH as shown in the above scheme, the Boc protecting group is selectively removed with trifluoracetic acid to provide the requisite free amino group.

The coupling of an ACOOH compound with a proline ester, is carried out by first protecting the amino group of the amino acid. Conventional amino protecting groups commonly used for temporary protection or blocking of the amino group are employed. Examples of such protecting groups include the alkoxy, alkenyloxy, cycloalkoxy, and aryloxycarbonyl groups such as ethoxycarbonyl, t-butyloxycarbonyl (Boc), cyclohexyloxycarbonyl, adamantyloxycarbonyl, trichloroethoxycarbonyl, benzyloxycarbonyl (Cbz), diphenylmethoxycarbonyl, and like groups. The ester group employed to protect the carboxy group of proline during the coupling reaction can be any of the commonly used readily removable ester groups such as t-butyl, benzyl, p-nitrobenzyl, p-methoxybenzyl, diphenylmethyl, trichloroethyl, phenacyl, or trialkylsilyl esters. In carrying out the coupling reaction one employs an ester group for proline which is removable by conditions under which the amino protecting group remains intact. The amino protecting group of the acylating acid ACOOH thus remains in place for protection of the amino group during the subsequent coupling with the arginine lactam compound to form c.

The perhydro bicyclo groups are prepared by hydrogenation of either the partially reduced or unsaturated acids by conventional procedures. For example, 1,2,3,4-tetrahydroisoquinoline-1-carboxylic acid is hydrogenated over platinum oxide in a solvent such as ethanol or acetic acid to provide the perhydro(decahydro) isoquinolin-1-carboxylic acid. The perhydro acids are then used as described above in the acylation of a proline ester. Examples of such perhydro derivatives represented by the formula 1 are N-(D-decahydroisoquinolin-1-carbonyl)-L-prolyl-L-arginine aldehyde and N-(D-decahydroisoquinolin-3-carbonyl)-L-prolyl-L-arginine.

The above described hydrogenation process provides a mixture of the cis and trans stereoisomers discussed hereinabove with the cis stereoisomers being formed in the greater amount. For example, compounds include D-1-(4aS, 8aS)-Piq-(L)-Pro-(L)-Arg-H, D-1-(4aR, 8aR)-Piq-(L)-Pro-(L)-Arg-H, D-1-(4aS, 8aR)-Piq-(L)-Pro-(L)-Arg-H, D-1-(4aR, 8aS)-Piq-(L)-Pro-(L)-Arg-H, D-3-(4aS, 8aS)-Piq-(L)-Pro-(L)-Arg-H, D-3-(4aR, 8aR)-Piq-(L)-Pro-(L)-Arg-H, D-3-(4aS, 8aR)-Piq-(L)-Pro-(L)-Arg-H, and D-3-(4aR, 8aS)-Piq-(L)-Arg-H.

The coupling reactions described above are carried out in the cold preferably at a temperature between about -20°C and about 15°C. The coupling reactions are carried out in an inert organic solvent such as dimethylformamide, dimethylacetamide, tetrahydrofuran, methylene chloride, chloroform, and like common solvents. Generally anhydrous conditions are used when, in the coupling reaction, an active ester of the acylating acid is used.

The compounds of the invention are isolated best in the form of acid addition salts. Salts of the compounds of formula 1 formed with acids such as those mentioned hereinabove useful as pharmaceutically acceptable salts for administration of the antithrombotic agents and for preparation of formulations of these agents. Other acid addition salts may be prepared and used in the isolation and purification of the peptides. For example, the salts formed with the sulfonic acids such as methanesulfonic acid, n-butanesulfonic acid, p-toluenesulfonic acid and naphthalene sulfonic acid may be so used.

A preferred method for isolating and purifying the compounds represented by the formula 1 while at the same time preparing a desired stable salt form is described in U.S. Patent 5,250,660 and involves preparative purification over C₁₈ reversed-phase chromatography. The aqueous phase comprises sulfuric acid or hydrochloric acid at a concentration between about 0.01% and about 0.05% and acetonitrile, THF, methanol or other suitable solvent serves as the organic component. The pH of the acidic eluant is adjusted to between about pH 4 and about pH 6, the exact pH being a function of the particular peptide, with a basic resin e.g. Bio-Rad AG-1X8 resin in the hydroxyl form. After pH adjustment the solution of the tripeptide salt e.g. sulfate or hydrochloride, is lyophilized to provide the purified salt dry powder form. In an example of the process crude 1-(1R, 4aR, 8aR)-Piq-L-Pro-L-Arg-H sulfate, contaminated with the epimeric D-Arg-H sulfate is dissolved in water and the solution is loaded on Vydac C₁₈ RPHPLC 5 cm X 50 cm column. A gradient of 2-20 percent B (A = 0.01 percent H₂SO₄; B = acetonitrile) over 10 hours is used. Multiple fractions are collected and those containing the desired product as determined by analytic RP-HPLC are pooled. The pH of the pooled fractions is adjusted to about pH 4.0 to about 4.5 with the Bio-Rad AG-1X8 resin in the hydroxyl cycle. After filtering the solution is lyophilized to provide pure 1-(1R, 4aR, 8aR)-Piq-L-Pro-L-Arg-H sulfate.

The compound of the invention is believed to selectively inhibit thrombin over other proteinases and nonenzyme proteins involved in blood coagulation without appreciable interference with the body's natural clot lysing ability (the compounds have a low inhibitory effect on fibrinolysis). Further, such selectivity is believed to permit use with thrombolytic agents' without substantial interference with thrombolysis and fibrinolysis.

The compound provided by the invention (formula 1) selectively inhibits the action of thrombin in man and animals (mammals). The inhibition of thrombin is demonstrated by in vitro inhibition of amidase activity of thrombin. The following Table 1 lists the apparent equilibrium constant (Kass) for interaction between the test compound (inhibitor and thrombin. The data in the table were obtained in an assay in which thrombin hydrolyzes the chromogenic substrate, N-benzoyl-D-phenylalanyl-L-valyl-L-arginyl-p-nitroanilide.

The assay was carried out in 50 µl buffer (0.03M Tris, 0.15M NaCl, pH 7.4) with 25 µl of human thrombin solution (purified human thrombin, Enzyme Research Laboratories, South Bend, Indiana, at 8 NIH units/ml) and 25 µl of test compound in a solvent (50% aqueous methanol (v:v)). Then 150 µl of an aqueous solution of the chromogenic substate (at 0.25 mg/ml) are added and the rates of hydrolysis of the substrate are measured by monitoring the reactions at 405 nm for the release of p-nitroaniline. Standard curves were constructed by plotting free thrombin concentration against hydrolysis rate. The hydrolysis rates observed with test compounds are then converted to "free thrombin" values in the respective assays by use of the standard curves. The bound thrombin (bound to test compound) was calculated by subtracting the amount of free thrombin observed in each assay from the known initial amount of thrombin observed in each assay. The amount of free inhibitor in each assay was calculated by subtracting the number of moles of bound thrombin from the number of moles of added inhibitor (test compound).

The Kass value is the hypothetical equilibrium constant for the reaction between thrombin and the test compound (I). $\text{Thrombin + I ⇄ Thrombin - I}$$\text{Kass =} \frac{\left[\text{Thrombin I}\right]}{\left[\left(\text{Thrombin}\right) \text{x} \left(\text{I}\right)\right]}$

Kass is calculated for a range of concentrations of test compounds and the mean value reported in units of liter per mole.

By substantially following the procedures described above for human thrombin, and using other human blood coagulation system serine proteases and using fibrinolytic system serine proteases, with the appropriate chromogenic substrates, identified below, the selectivity of the compound of the present invention with respect to the coagulation factor serine proteases and to the fibronolytic serine proteases are evaluated as well as their substantial lack of interference with human plasma clot fibrinolysis.

Human factor Xa is purchased from Enzyme Research Laboratories, South Bend, Indiana. Chromogenic substrate: N-Benzoyl-Ile-Glu-Gly-Arg-p-nitroanilide (for factor Xa) is purchased from KabiVitrum, Stockholm, Sweden, or from Midwest Biotech, Fishers, Indiana. Bovine trypsin is purchased from Worthington Biochemicals, Freehold, New Jersey. Chromogenic substrate, N-Benzoyl-Phe-Val-Arg-p-nitroanilide, the substrate for human thrombin and for trypsin, is synthesized according to procedures described above for the compound of the present invention, using known methods of peptide coupling from commercially available reactants, or purchaed from Midwest Biotech, Fishers, Indiana.

Human plasmin is purchased from Boehringer Mannheim, Indianapolis, Indiana; nt-PA is purchased as single chain activity reference from American Diagnostica, Greenwich, Connecticut. Plasmin chromogenic substrate H-D-Val-Leu-Lys-p-nitroanilide and tissue plasminogen activator (t-PA) substrate H-D-Ile-Pro-Arg-p-nitroanilide are purchased from Kabi Vitrum, Stockholm, Sweden.

In the chromogenic substrates described above the three-letter symbols Ile, Glu, Gly, Pro, Arg, Phe, Val, Leu and Lys are used to indicate the corresponding amino acid group isoleucine, glutamic acid, glycine, proline, arginine, phenylalanine, valine, leucine and lysine, respectively.

Table 1 which follows lists the Kass values obtained with the indicated compound.

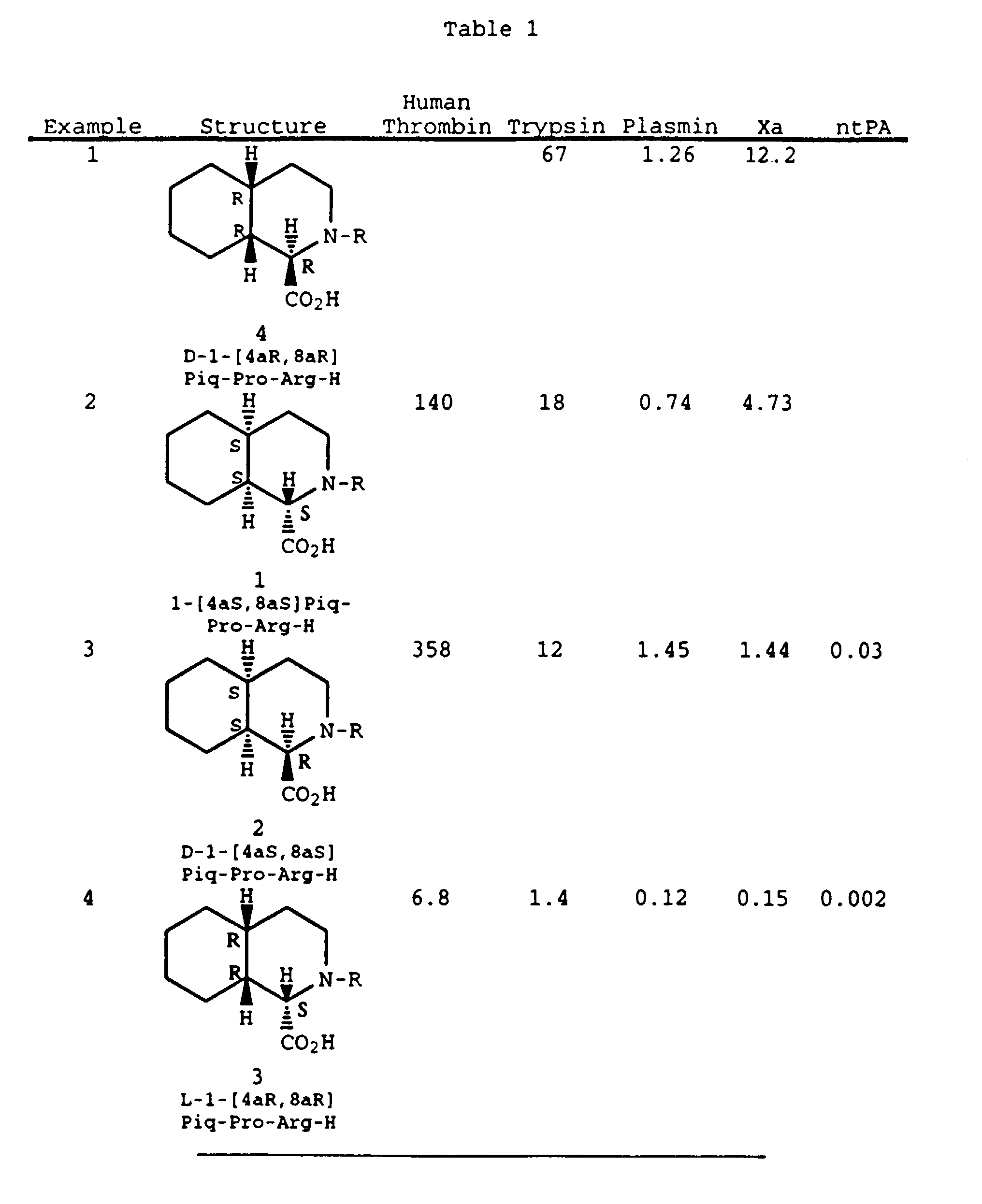

The compound of the invention selectively inhibits clot formation without appreciable interference with the bodies natural clot lysing ability e.g. the compounds have a low inhibitory effect on fibrinolysis.

The invention in one of its aspects provides a method for inhibiting the formation of blood clots in man and animals (mammals) which comprises administering to said man or animal an effective clot inhibiting non-toxic dose of a compound represented by the formula 1. The anti-coagulant compound is administered orally, parenterally e.g. by intravenous infusion (iv), intramuscular injection (im) or subcutaneously (sc).

An effective clot inhibiting dose is between about 5 mg and about 1000 mg. The dose regime may vary e.g. for prophylactic use a single daily dose may be administered or multiple doses such as 3 or 5 times daily may be appropriate. In critical care situations a compound of the invention is administered by iv infusion at a rate between about 0.1 mg/kg/h and about 1.0 mg/kg/h.

For oral administration a dose of a compound of the instant invention in the range of about 0.1 mg/kg to about 20 mg/kg can be administered one or more times per 24 hours. Preferably the dose is in the range of about 0.5 mg/kg to about 5 mg/kg and is administered up to 4 times per 24 hours. The instant compounds can be orally administered using standard forms such as tablets, capsules, and the like as provided below.

The method of this invention also is practiced in conjunction with a clot lysing agent e.g. tissue plasminogen activator (tPA), modified tPA, streptokinase or urokinase. In cases when clot formation has occurred and an artery or vein is blocked, either partially or totally, a clot lysing agent is usually employed. A compound of the invention can be administered along with the lysing agent or subsequent to its use to prevent the reoccurrence of clot formation.

This invention also provides pharmaceutical formulations for use in the above described therapeutic method. Pharmaceutical formulations of the invention comprise an effective thrombin inhibiting amount of a compound of formula I in association with a pharmaceutically acceptable carrier, excipient or diluent. For oral administration the antithrombotic compound is formulated in gelatin capsules or tablets which may contain excipients such as binders, lubricants, disintegration agents and the like. For parenteral administration the antithrombotic is formulated in a pharmaceutically acceptable diluent e.g. physiological saline (0.9 percent), 5 percent dextrose, Ringer's solution and the like.

The compound of the present invention can be formulated in unit dosage formulations comprising a dose between about 0.1 mg and about 1000 mg. Preferably the compound is in the form of a pharmaceutically acceptable salt such as for example the sulfate salt, acetate salt or a phosphate salt. An example of a unit dosage formulation comprises 5 mg of a compound of the present invention as a pharmaceutically acceptable salt in a 10 ml sterile glass ampoule. Another example of a unit dosage formulation comprises about 10 mg of a compound of the present invention as a pharmaceutically acceptable salt in 20 ml of isotonic saline contained in a sterile ampoule.

The compounds can be administered by a variety of routes including oral, rectal, transdermal, subcutaneous, intravenous, intramuscular, and intranasal. The compounds of the present invention are preferably formulated prior to administration. Another embodiment of the present invention is a pharmaceutical formulation comprising an effective amount of a compound of Formula I or a pharmaceutically acceptable salt or solvate thereof in association with a pharmaceutically acceptable carrier, diluent or excipient therefor.

The active ingredient in such formulations comprises from 0.1 percent to 99.9 percent by weight of the formulation. By "pharmaceutically acceptable" it is meant the carrier, diluent or excipient must be compatible with the other ingredients of the formulation and not deleterious to the recipient thereof.

The present pharmaceutical formulations are prepared by known procedures using well known and readily available ingredients. The compositions of this invention may be formulated so as to provide quick, sustained, or delayed release of the active ingredient after administration to the patient by employing procedures well known in the art. In making the compositions of the present invention, the active ingredient will usually be admixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosols, (as a solid or in a liquid medium), soft and hard gelatin capsules, suppositories, sterile injectable solutions, sterile packaged powders, and the like.

The following formulation examples are illustrative only and are not intended to limit the scope of the invention in any way. "Active ingredient," of course, means a compound according to Formula I or a pharmaceutically acceptable salt or solvate thereof.

### Formulation 1

Hard gelatin capsules are prepared using the following ingredients:

| | Quantity (mg/capsule) |
|---|---|
| Active ingredient | 250 |
| Starch, dried | 200 |
| Magnesium stearate | 10 |
| Total | $\overline{\text{460 mg}}$ |

### Formulation 2

A tablet is prepared using the ingredients below:

| | Quantity (mg/capsule) |
|---|---|
| Active ingredient | 250 |
| Cellulose, microcrystalline | 400 |
| Silicon dioxide, fumed | 10 |
| Stearic acid | 5 |
| Total | $\overline{\text{665 mg}}$ |

The components are blended and compressed to form tablets each weighing 665 mg

### Formulation 3

An aerosol solution is prepared containing the following components:

| | Weight |
|---|---|
| Active ingredient | 0.25 |
| Ethanol | 25.75 |
| Propellant 22 (Chlorodifluoromethane) | 70.00 |
| Total | $\overline{\text{100.00}}$ |

The active compound is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

### Formulation 4

Tablets, each containing 60 mg of active ingredient, are made as follows:

| | |
|---|---|
| Active ingredient | 60 mg |
| Starch | 45 mg |
| Microcrystalline cellulose | 35 mg |
| Polyvinylpyrrolidone (as 10 % solution in water) | 4 mg |
| Sodium carboxymethyl starch | 4.5 mg |
| Magnesium stearate | 0.5 mg |
| Talc | 1 mg |
| Total | $\overline{\text{150 mg}}$ |

The active ingredient, starch and cellulose are passed through a No. 45 mesh U.S. sieve and mixed thoroughly. The aqueous solution containing polyvinyl- pyrrolidone is mixed with the resultant powder, and the mixture then is passed through a No. 14 mesh U.S. sieve. The granules so produced are dried at 50°C and passed through a No. 18 mesh U.S. Sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

### Formulation 5

Capsules, each containing 80 mg of active ingredient, are made as follows:

| | |
|---|---|
| Active ingredient | 80 mg |
| Starch | 59 mg |
| Microcrystalline cellulose | 59 mg |
| Magnesium stearate | 2 mg |
| Total | $\overline{\text{200 mg}}$ |

The active ingredient, cellulose, starch, and magnesium stearate are blended, passed through a No. 45 mesh U.S. sieve, and filled into hard gelatin capsules in 200 mg quantities.

### Formulation 6

Suppositories, each containing 225 mg of active ingredient, are made as follows:

| | |
|---|---|
| Active ingredient | 225 mg |
| Saturated fatty acid glycerides | 2,000 mg |
| Total | $\overline{\text{2,225 mg}}$ |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

### Formulation 7

Suspensions, each containing 50 mg of active ingredient per 5 ml dose, are made as follows:

| | |
|---|---|
| Active ingredient | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to total | 5 ml |

The active ingredient is passed through a No. 45 mesh U.S. sieve and mixed with the sodium carboxymethyl cellulose and syrup to form a smooth paste. The benzoic acid solution, flavor and color are diluted with a portion of the water and added, with stirring. Sufficient water is then added to produce the required volume.

### Formulation 8

An intravenous formulation may be prepared as follows:

| | |
|---|---|
| Active ingredient | 100 mg |
| Isotonic saline | 1,000 ml |

The solution of the above ingredients generally is administered intravenously to a subject at a rate of 1 ml per minute.

An example of a unit dosage formulation comprises 5 mg of (1R, 4aR, 8aR)-perhydroisoquinolin-1-carbonyl-L-prolyl-L-arginine aldehyde sulfate salt in a 10 ml sterile glass ampoule. Another example of a unit dosage formulation comprises about 10 mg of (1R, 4aR, 8aR)-perhydroisoquinolin-1-carbonyl-L-prolyl-L-arginine aldehyde sulfate in 20 ml of isotonic saline contained in a sterile ampoule.

A preferred formulation is a unit dosage form comprising between 5 mg and 50 mg of (1R, 4aR, 8aR)-perhydroisoquinolin-1-carbonyl)-L-prolyl-L-arginine aldehyde sulfate in sterile ampoules.

The following Examples are provided to further describe the invention and are not to be construed as limitations thereof.

The Rf values in the following examples were determined by silica gel thin layer chromatography using Kieselgel 60F-254 (Merck, Darmstradt) in the following solvent systems:
(A) Chloroform-methanol-acetic acid, 135:15:1, v:v:v
(B) ethyl acetate-acetic acid-absolute ethanol, 90:10:10, v:v:v
(C) chloroform-methanol-acetic acid, 90:30:5, v:v:v
(D) ethyl acetate-hexanes, 30:70, v:v

The analytical HPLC methods used in the examples were as follows:

### Method 1.

Waters 600 E using a Vydac C18 reversed-phase column of 0.46 cm x 10 cm. The chromatogram was monitored on a LDC at 220 nM using a gradient of A = 0.01M ammonium acetate and B = acetonitrile.

### Method 2.

Pharmacia FPLC using a PepRPC measuring 0.5 cm x 5.0 cm. Monitoring was done on a Pharmacia UV-M at 214 nM using a gradient of either A = 0.01M ammonium acetate or B = acetonitrile.

The abbreviations used herein have the following meanings.

Amino acids: Arg = arginine, Pro = proline, Phg = phenylglycine
Boc = t-butyloxycarbonyl
Bzl = benzyl
Cbz (or z) = benzyloxycarbonyl
DCC = dicyclohexylcarbodiimide
DMF = dimethylformamide
DMSO = dimethylsulfoxide
FAB-MS = fast atom bombardment mass spectrum
FD-MS = field desorption mass spectrum
THF = tetrahydrofuran
TLC = thin layer chromatography

### Example 1

### Preparation of (1R, 4aR, 8aR)-perhydroisoquinolin-1-carbonyl-L-Prolyl-L-Arginine aldehyde

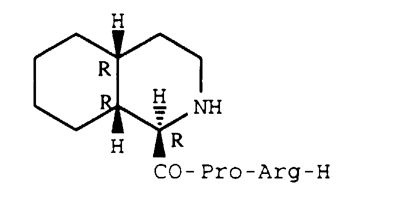

### Methylcarbamate-phenethylamine (1)

To a stirred solution of phenethylamine (75.2 mL, 0.6 mol) and triethylamine (83 mL, 0.6 mol) in THF (500 mL) was added slowly methyl chloroformate (46.2 mL, 0.6 mol) dissolved in THF (50 mL). After the reaction was stirred for an additional 1 h at room temperature, diethyl ether (2 L) and 1 N HCl (800 mL) was added. The organic layer was washed with water, dried (MgSO₄), filtered, and the filtrate was concentrated *in vacuo* to give a clear oil of pure title compound (102 g, 95%).

### Methylcarbamate-DL-1,2,3,4-tetrahydoisoquinoline-1-carboxylic acid (2)

To a solution of methylcarbamate-phenethylamine (1) (102 g, 0.57 mol) in trifluoroacetic acid (300 mL) was added glyoxylic acid (63 g, 0.68 mol) and heated to reflux temperature. After 4 h at reflux the reaction was cooled to room temperature, solvent removed *in vacuo*, and diethyl ether (800mL) / water (100 mL) was added to the residue. The reaction mixture pH was raised to 12 with 5 N NaOH and the aqueous layer separated. To the aqueous layer was added diethyl ether (500 mL), and the solution was acidified to pH 2.5 with 5 N HCl. The organic layer was separated, dried (MgSO₄), filtered, and the filtrate was concentrated *in vacuo* to afford an oil of pure title compound (107 g,.80%); FAB-MS 236 (MH⁺).

### Methylcarbamate-DL-1,2,3,4-tetrahydoisoquinoline-1-carboxylic-t-butyl ester (3)

To a stirred, cooled (0 °C), solution of methylcarbamate-DL-1,2,3,4-tetrahydoisoquinoline-1-carboxylic acid (2) (105 g, 0.45 mol) in CH₂Cl₂ (200 mL) was added t-butanol (52 mL, 0.54 mol), 4-dimethyl amino pyridine (10g; 0.08 mol) and DCC (92 g, 0.45 mol). After 2 h at 0 °C and 24 h at room temperature, the solvent was removed in vacuo, and ethyl acetate (800mL) / 1 N NaHCO₃ (300 mL) was added to the residue. The organic layer was separated, washed sequentially with water, 1.5 N citric acid, and water. The organic layer was dried (MgSO₄), filtered, and the filtrate was concentrated in vacuo to afford an oil of pure title compound (106 g,.81%); FAB-MS 292 (MH⁺); TLC R_{f} (A) 0.61; elemental analysis (calcd) C₁₆H₂₁NO₄: C, 65.96; H, 7.27; N, 4.81. Found: C, 66.24, H, 7.28, N, 4.73.

### Methylcarbamate-DL-1,2,3,4,6,7,8-perhydoisoquinoline-1-carboxylic-t-butyl ester (4)

A solution of methylcarbamate-DL-1,2,3,4-tetrahydoisoquinoline-1-carboxylic-t-butyl ester (3) (105 g, 0.36 mol) in t-butanol (800 mL) was reacted with 5% Rh / Al₂O₃ (52.5 g) at 800 psi with hydrogen in a high pressure apparatus at 50 °C for 24 hours. The reaction mixture was filtered through a Celite® pad, and the filtrate was concentrated *in vacuo.* The resulting oil was dried to give pure title compound (96.5 g, 90%) FD-MS 298 (MH⁺); TLC R_{f} (C) 0.63.

### Methylcarbamate-1,2,3,4,4aS,6,7,8,8aS-perhydoisoquinoline-S-1-carboxylic ethyl ester + Methylcarbamate-1,2,3,4,4aR,6,7,8,8aR-perhydoisoquinoline-R-1-carboxylic ethyl ester (5)

To a solution of methylcarbamate-DL-1,2,3,4,6,7,8-perhydoisoquinoline-1-carboxylic-t-butyl ester (4) (81.2 g, 273 mmol) in EtOH (500 mL) was added sodium ethoxide (21% in ethanol) (88.4 mL, 273 mmol) and the reaction was refluxed (24 h). The organic solvent was evaporated *in vacuo*, ethylacetate (400mL) and water (100 mL) was added to the residue. The organic layer was separated, washed twice with water, dried (MgSO₄), filtered, and the filtrate was concentrated *in vacuo* to afford an oil of pure title compounds (70 g,.95%); FAB-MS 270 (MH⁺); TLC R_{f} (A) 0.61.

### Methylcarbamate-1,2,3,4,4aS,6,7,8,8aS-perhydoisoquinoline-S-1-carboxylic acid + Methylcarbamate-1,2,3,4,4aR,6,7,8,8aR-perhydoisoquinoline-R-1-carboxylic acid (6)

To a solution of 5 (70 g, 260 mmol) in THF (250 mL) was added 2 N NaOH (156 mL, 312 mmol) and the reaction stirred at room temperature (30 h). The organic solvent was evaporated *in vacuo*, diethyl ether (400mL) and water (100 mL) was added to the residue. The aqueous layer separated and ethyl acetate (400 mL) was added. The pH of the solution was adjusted to 2.0 with 5 N HCl. The organic layer was dried (MgSO₄), filtered, and the filtrate was concentrated *in vacuo* to give a clear oil. The oil was crystallized from hexane (200 mL) to afford pure title compound (46.4 g,.74%); FAB-MS 242 (MH⁺); TLC R_{f} (A) 0.36; elemental analysis (calcd) C₁₂H₁₉NO₄: C, 59.74; H, 7.94; N, 5.81. Found: C, 59.95, H, 7.88, N, 5.54.

### Cbz-1,2,3,4,4aS,6,7,8,8aS-perhydoisoquinoline-S-1-carboxylic acid + Cbz-1,2,3,4,4aR,6,7,8,8aR-perhydoisoquinoline-R-1-carboxylic acid (7)

To a stirred solution of 6 (46 g, 191 mmol), at room temperature, in anhydrous CH₃CN (200 mL) under an inert atmosphere was added a solution of iodotrimethyl silane (62.4 mL, 440 mmol) in CH₃CN (60 mL). The reaction was stirred at 55 °C for 30 min. and cooled to room temperature. The reaction was quenched with water (100 mL) followed by sodium metabisulfite (1 The pH of the reaction was raised to 10.0 with 5 N NaOH and benzyl chloroformate (27.3 mL, 191 mmol) was added dropwise while the pH maintained at 10 with 2 N NaOH. After the reaction was stirred for an additional 30 min. at room temperature the organic solvent was evaporated *in vacuo*, and diethyl ether (200 mL) was added. The reaction was allowed to stand at room temperature (2 h) and ethyl acetate (200 mL) was added. The aqueous solution was acidified to pH 2.5 with 5 N HCl the organic layer was separated, dried (MgSO₄), filtered, and the filtrate was concentrated *in vacuo* to give pure title compound as an oil (39.5 g, 65%); FAB-MS 318 (MH⁺); elemental analysis (calcd) C₁₈H₂₃NO₄: C, 68.12; H, 7.30; N, 4.41. Found: C, 66.37, H, 7.52, N, 4.37.

### Cbz-1,2,3,4,4aS,6,7,8,8aS-perhydoisoquinoline-S-1-carbonyl-Pro-t-Bu + Cbz-1,2,3,4,4aR,6,7,8,8aR-perhydoisoquinoline-R-1-carbonyl-Pro-t-Bu (8)

To a stirred, cooled (0 °C) solution of 7 (39 g, 123 mmol) in DMF (200 mL) was added Proline-t-butylester (21.1 g, 123 mmol), 1-hydroxybenzotriazole (16.6 g, 123 mmol), and DCC (25.3 g, 123 mmol). The reaction was stirred for 2 h at O °C and 24 h at room temperature. The reaction precipitate was filtered and the filtrate concentrated *in vacuo* to an oil. The oil was dissolved in EtOAc (200 mL) and water (100 mL). The organic layer was washed sequentially with 1 N NaHCO₃, water, 1.5 N citric acid, and water. The organic layer was dried (MgS0₄), filtered, and the filtrate evaporated to an amorphous solid of the title compound (52.7 g, 91%) FAB-MS 471 (MH⁺).

### Cbz-1,2,3,4,4aR,6,7,8,8aR-perhydoisoquinoline-(1R)-carbonyl-Pro-OH (9)

To a stirred solution of 8 (52.4 g, 111 mmol) in CH₂Cl₂ (20 mL) was added trifluoroacetic acid (70 ml) and anisole (5 ml). The reaction was stirred at room temperature for 1 h and concentrated *in vacuo* without heating. The reaction was diluted with diethyl ether (400 ml), water (100 mL), and the pH of the solution was adjusted to 10.0 with 5 N NaOH. The aqueous layer separated and ethyl acetate (300 mL) was added. The pH of the solution was adjusted to 2.5 with 5 N HCl. The organic layer was separated, dried (MgSO₄), filtered, and the filtrate was concentrated *in vacuo* to give a clear oil. The oil was dissolved in diethylether (500 mL) and L(-) alphamethylbenzylamine was added to the solution. The solution was allowed to stand at room temperature (24 h). The resulting solid was filtered washed with diethyl ether and dried. The solid was suspended in ethyl acetate, washed with 1.5 N citric acid, and water. The organic layer was dried (MgS0₄), filtered, and the filtrate evaporated to give the title compound as an oil (20.2 g, 44%) FAB-MS 415 (MH⁺); [α]_{D} = 3.2° (C, 0.5 MeOH); elemental analysis (calcd) C₂₃H₃₀N₂O₅: C, 66.65; H, 7.30; N, 6.76. Found: C, 66.38, H, 7.36, N, 6.63.

### (A) Boc-L-Arg(Cbz)-OH

N-Boc-arginine hydrochloride (Boc-Arg-OH·HCl). (82.1 g, 250 mmole) was dissolved in 240 ml of 5N NaOH in a 3-necked round bottom flask. The solution was cooled to -5°C and benzyl chloroformate (143 ml, 1.0 mole, 4 eq.) was added dropwise over 55 min while the pH of the mixture was maintained at 13.2-13.5 with 5N NaOH (250 ml). The reaction mixture was stirred for one hour at -5°C after addition of the chloroformate was completed. The reaction mixture was diluted with 100 ml of water and 500 ml of diethyl ether and the aqueous layer was separated and extracted twice with 40 ml portions of diethyl ether. The aqueous layer was acidified to pH 3.0 with 3N H₂SO₄ (560 ml) and extracted with 550 ml of ethyl acetate. The separated aqueous layer was extracted once with ethyl acetate and the extract combined with the previous ethyl acetate extract. The combined extracts were washed with water, dried over MgSO₄ and evaporated to dryness under vacuum. The residue was triturated with ether and the precipitated produce was filtered and dried. There were obtained 66.1 g of (3) Boc-Arg(Cbz)-OH (65% of theory): TLC R_{f} (C) 0.43; FD-MS 408 (M⁺).
¹H NMR (CDCl₃), δ 1.42 (s, 9H), 1.61-1.91 (m, 4H), 3.23-3.41 (m, 2H), 4.17 (d, 1H), 5.21 (s, 2H), 5.62 (d, 1H), 7.30-7.42 (m, 6H), 8.37 (m, 1H),

### (B) Boc-Arg(Cbz)-Lactam

A solution of Boc-Arg(Cbz)-OH (A) prepared as described abve (66.0 g, 0.162 mole) in 230 ml of dry THF was cooled to -10°C in an ice-acetone bath. To the cold solution was added N-methylmorpholine (18.7 ml, 1.05 eq) followed by isobutyl chloroformate (22.5 ml, 1.05 eq) and the mixture was stirred for 5 minutes at -10°C. Next, triethylamine (23.5 ml, 1.05 eq) was added and the mixture was stirred for 1 h at -10°C and at room temperature for 1 h. The reaction mixture was poured into one liter of an ice-water mixture and the title compound precipitated. The precipitate was filtered, washed with cold water, dried under vacuum, and was crystallized from ethyl acetate. There was obtained 38.05 a (60% of theory) of the title compound, Boc-Arg(Cbz)-lactam: TLC R_{f} (A) 0.77; FD-MS 291 (MH⁺).
¹H NMR (CDCl₃), δ 1.48 (s, 9H), 1.78-1.98 (m, 2H), 2.50 (m, 1H), 3.41 (m, 1H), 4.43 (m, 1H), 4.90 (m, 1H), 4.16 (s, 2H), 5.27 (m, 1H), 7.28-7.45 (m, 6H), 9.41 (m, 1H), 9.68 (m, 1H).

### Cbz-1,2,3,4,4aR,6,7,8,8aR-perhydoisoquinoline-R-1-carbonyl-Pro-Arg(Cbz) lactam (11)

In flask 1 compound 9 (13.9 g, 33.5 mmole) was dissolved in DMF (50 ml), cooled to -15 °C, and N-methylmorpholine (3.7 ml, 33.5 mmole) was added followed by isobutylchloroformate (4.4 ml, 33.5 mmole). The reaction mixture was stirred at -15 °C for 1 min. In flask 2 HCl·Arg(Cbz)-Lactam (10.9 g, 33.5 mmole) was dissolved in DMF (50 ml), cooled to 0 °C, and diisopropylethylamine (14.6 ml, 83.8 mmole) was added to the solution. The reaction mixture was stirred at 0 °C for 1 min. The contents of flask 2 was added to flask 1, and the reaction mixture was stirred for 2 h (-15 °C) followed by 24 h at room temperature. To the reaction was added 1 N NaHCO₃ (10 ml) and the reaction solvent was removed *in vacuo* to an oil. The residue was dissolved in EtOAc (200 ml) and washed sequentially with 1.5 N citric acid, water, 1 N NaHCO₃ (100 ml), and water. The organic solution was dried (MgSO₄), filtered, and concentrated to dryness *in vacuo* to give a crude solid. The crude solid was purified by chromatography on silica gel using a step gradient elution (hexane 100 to hexane-EtOAc 30:70) to yield pure compound 10.(9.0 g, 39%): FAB-MS 687 (MH⁺); elemental analysis (calcd) C₃₇H₄₆N₆O₇: C, 64.71; H, 6.75; N, 12.24. Found: C, 64.23, H, 6.69, N, 11.88.

### Cbz-1,2,3,4,4aR,6,7,8,8aR-perhydoisoquinoline-R-1-carbonyl-Pro-Arg(Cbz)-H (12)

To a stirred, cooled (-70 °C) solution of 11 (9.0 g, 13.1 mmol) under a nitrogen atmosphere in anhydrous THF (100 mL) was added lithium aluminum hydride 1 M in THF (13.1 mL, 13.1 mmol). The reaction was stirred for 30 min at -70 °C. A solution of 5 mL of THF and 5 mL of 0.5 N H₂S0₄ was added dropwise to the reaction. The reaction was diluted with EtOAc (175 mL) and water (100 mL). The organic layer was separated, dried (MgSO₄), and filtered. The organic solvent was removed *in vacuo* to to give an amorphous solid of the title compound (8.2 g, 91%): FAB-MS 689 (MH⁺).
1,2,3,4,4aR,6,7,8,8aR-perhydoisoquinoline-R-1-carbonyl-Pro-Arg-H·H₂SO₄ (12)

Compound 12 (8.2 g.11.9 mmol) dissolved in ethanol (50 mL), water (10 mL), and 1 N H₂SO₄ (30 mL, 29.7 mmol) was hydrogenated in the presence of 5% Pd/C catalyst (4.0 g) at ambient temperature and pressure. After the reaction was completed, the catalyst was removed by filtration. The filtrate concentrated down to 40 mL *in vacuo* and water (50 mL) was added. The pH of the solution was adjusted to 4.2 with BioRad AG1-X8 resin (hydroxide form). The resin was removed by filtration and the solution lyophilized to give crude title compound (5.46 g). The solid (5.46 g) was dissolved in 0.01% H₂SO₄ and applied to a 5 X 25 cm column Vydac C₁₈ resin. A gradient of increasing concentrations of CH₃CN (1% to 5%) was used to elute the peptide from the column. Fractions were collected and pooled on the basis of analytical RP-HPLC profile. The combined fractions were adjusted to pH 4.2 using AG1-X8 resin (Bio-Rad analytical anion exchange resin 50-100 mesh) in hydroxide form. The solution was filtered, and the filtrate was lyophilized to afford pure title compound (2.4 g, 39%): FAB-MS 421 (MH⁺); [α]_{D} = -102.8° (C, 0.5 / 0.01 N H₂SO₄); elemental analysis (calcd) C₂₁H₃₆N₆O₃·H₂SO₄·2H₂O: C, 45.47; H, 7.63; N, 15.16. Found: C, 45.05, H, 7.44, N, 15.02.

## Claims

1. A compound of the formula 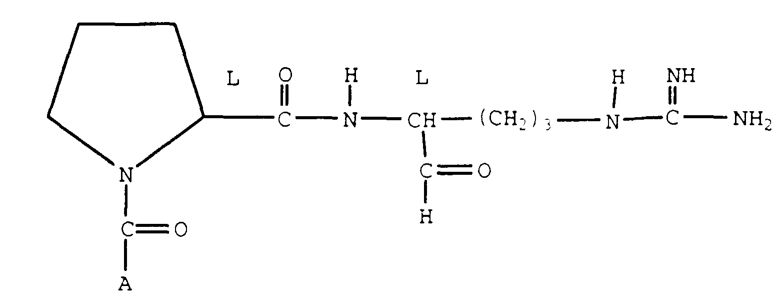 wherein A is 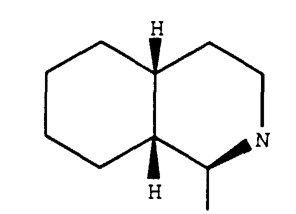 and pharmaceutically acceptable salts and solvates thereof.

2. A sulfate salt of the compound of claim 1.

3. A compound as claimed in any of claims 1 to 2 for use as an antithrombotic agent.

4. A pharmaceutical formulation comprising as an active ingredient a compound as claimed in any one of claims 1 to 2 associated with one or more pharmaceutically acceptable carriers, excipients or diluents therefor.

## Patentansprüche

1. Verbindung der Formel 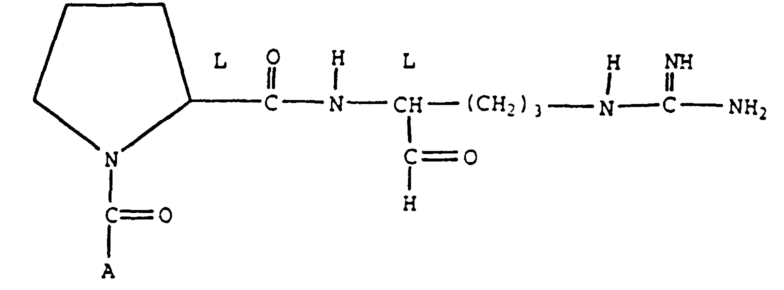 worin A steht für 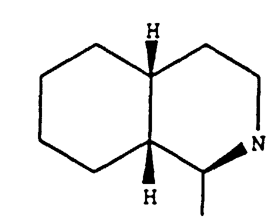 und pharmazeutisch annehmbare Salze und Solvate hiervon.

2. Sulfatsalz der Verbindung nach Anspruch 1.

3. Verbindung nach einem der Ansprüche 1 bis 2 zur Verwendung als antithrombotisches Mittel.

4. Pharmazeutische Formulierung, die als Wirkstoff eine Verbindung nach einem der Ansprüche 1 bis 2 zusammen mit einem oder mehreren pharmazeutisch annehmbaren Trägern, Hilfsstoffen oder Verdünnungsmitteln hierfür enthält.

## Revendications

1. Composé répondant à la formule 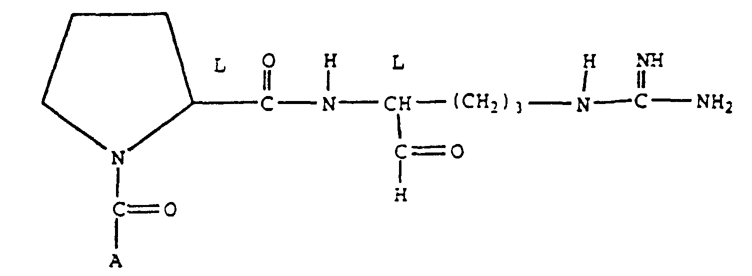 dans laquelle A représente 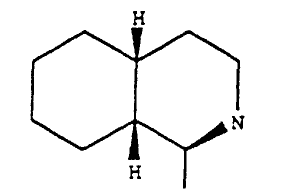 et les sels et solvates pharmaceutiquement acceptables de celui-ci.

2. Sel de sulfate du composé selon la revendication 1.

3. Composé selon l'une quelconque des revendications 1 à 2 pour l'utilisation comme agent antithrombotique.

4. Formulation pharmaceutique comprenant comme ingrédient actif un composé selon l'une quelconque des revendications 1 à 2, associé à un ou davantage de supports, excipients ou diluants pharmaceutiquement acceptables pour celui-ci.
